# EUROPEAN PATENT SPECIFICATION

(11) **EP 3 213 645 B1**
(45) Date of publication and mention of the grant of the patent: **24.10.2018**
(21) Application number: 17159012.8
(22) Date of filing: 02.03.2017
(51) Int. Cl.: A23L 33/105, A21D 13/06, A61K 36/63, A23L 19/00, A21D 2/36

(54) **PRODUCTION OF AN OLIVE-BASED NUTRACEUTICAL FOOD PRODUCT**
HERSTELLUNGS EINES AUF OLIVEN BASIERENDEN NUTRAZEUTISCHES LEBENSMITTELS
PRODUCTION D'UN PRODUIT NUTRACEUTIQUE A BASE D'OLIVE

(30) Priority: 03.03.2016 EP 16158560
(43) Date of publication of application: 06.09.2017
(73) Proprietor: OLIOCRU' S.r.l., 38062 Arco (TN) (IT)
(72) Inventor: CONTERNO, Lorenza, 38062 Arco (TN) (IT); MARTINELLI, Francesca, 38062 Arco (TN) (IT); MORANDINI, Marco, 38062 Arco (TN) (IT); TAMBURINI, Matteo, 38062 Arco (TN) (IT)
(74) Representative: Laghi, Alberto

(56) References cited:
- EP-A1- 1 922 933
- WO-A1-00/04794
- WO-A1-2007/042742
- WO-A1-2010/061412
- WO-A1-2013/030426
- US-B1- 6 309 652

## Description

The present invention relates to a process for the production of a food product.

In particular, the present invention is to be used as advantage for the food industry to produce a functional (nutraceutical) food product through the processing of a food grade paste of olives or equivalent one seed fruits, to which the description hereafter will make explicit reference without thereby losing in generality.

As it is known, the olives contain a number of substances, such as polyphenols, tocopherols, sterols, squalene, etc., which have been shown to play a very important and significant role for human health.

In general, only a small percentage of these substances is transferred to the oil during the olive oil production

In fact, the polyphenols, substances with high antioxidant capacity, represent the 1-2% of the fresh weight of the olive, while in the oil it is possible to find about the 0.1% at most; fibres naturally present in the olive fruit are not transferred to the oil because of their not fat soluble nature.

The preparation of dry dehydrated and defatted olive pulp powder is described in WO2013/030426. The preparation of bakery products using mixtures of flour with an antioxidant such as an extract from olive pulp is disclosed in WO2010/061412.

The purpose of the present invention, which is defined by the claims, is therefore to provide a process for the production of a food product with both optimum functional and organoleptic characteristics, with high content of natural fiber and polyphenols.

The functional characteristics of the present invention and its advantages over the state of the art, will be highlighted and clarified after the claims hereafter, and in particular after the examination of the description, referring to the flow chart (Figure A) showing the preferred, but not the limiting variant, of the production process of the functional food in question.

The starting raw material is a given amount of olives collected in the optimal seasonal period (depending on the variety) arranged to obtain the necessary amount of polyphenols (determined through in vivo study).

The whole olives are ground by crushing or, preferably, by destoning, to obtain a food grade paste of milled olives.

In particular, it has to be highlighted that destoning, unlike the most common process in which the olive pulp is ground together with the pit (crushing), effectively allows to obtain olive paste of higher quality, free of those enzymes present in the seeds and released into the paste from the broken pits. These enzymes are, together with the oxygen of the air, degradation agents of the paste polyphenols (oxidation of polyphenols).

Moreover, the absence of pit fragments, otherwise generated during crushing, makes the paste suitable for the human consumption without other treatment. In the next step, the olive paste is separated from the oil whose presence would lead to both a technological problem in the later step of drying and a higher unwanted energy content in the final product.

To allow the olive paste thus obtained to be used as nutraceutical food, such olive paste is to be subjected with a drying step and then be ground to obtain a powder. According to the flow chart in the attached diagram, the drying process can be performed with two different and alternative technological phases:
- Variant indicated with (I), to the left in the diagram: mixing phase of the olive paste with a blend of different vegetable flours and subsequently a drying step. Preferably, the drying process of the mixture takes place at low temperature (less than 60°C) under vacuum, or by other technologies (for example without vacuum and at temperatures higher than 60°C) to avoid oxidation during the drying phase. The final product must then be ground and sieved to avert the risk of accidental presence of pit fragments.
- Variant indicated with (II), to the right of the diagram: drying phase of the olive paste, where the drying of the paste takes place at low temperature under vacuum (less than 60°C) or by microwave or by exploiting other technologies (for example without vacuum and at temperatures higher than 60°C) to avoid oxidation during the drying process. The olive flour thus obtained can be mixed with a specific blend of different vegetable flours. The final product must then be ground and sieved to avert the risk of accidental presence of pit fragments.

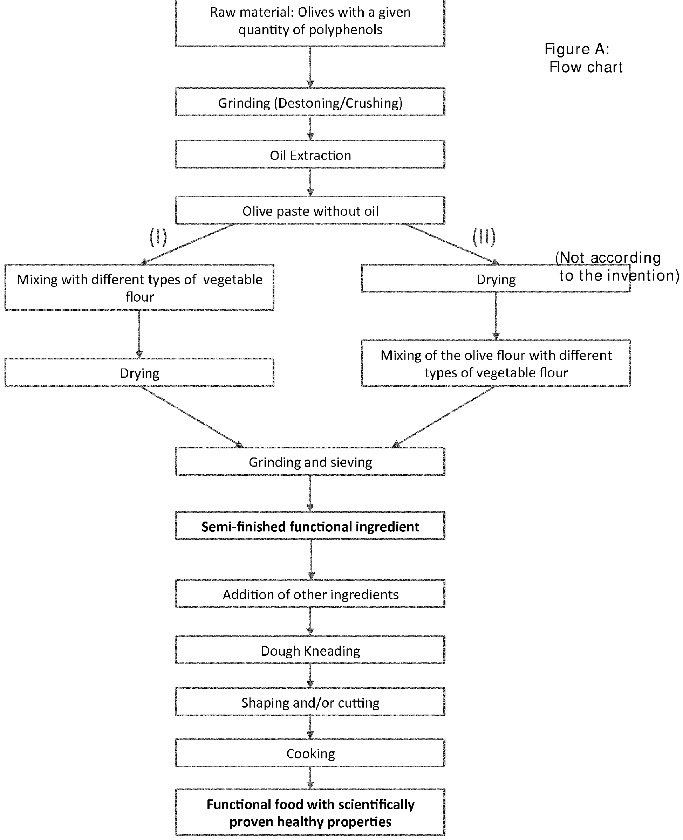

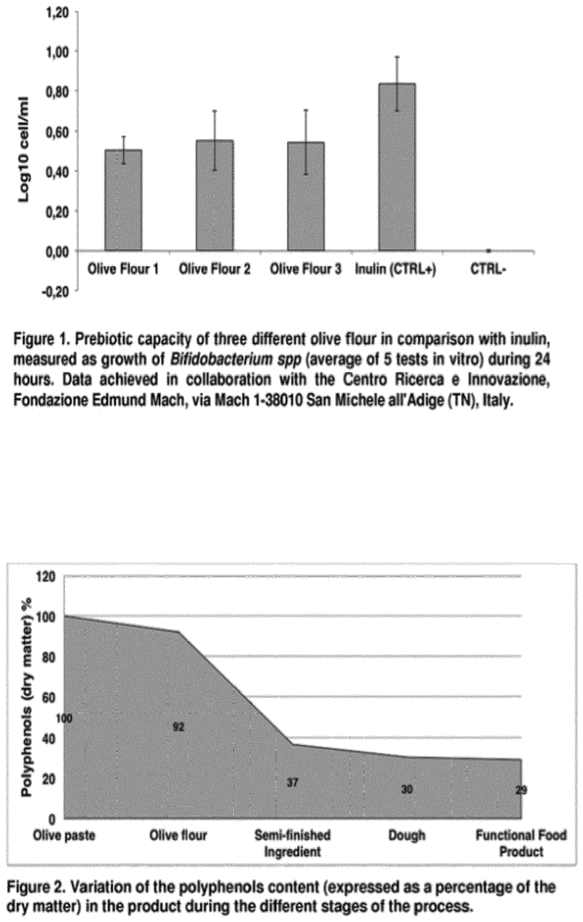

That variant (II) is not part of the present invention, as is provided for information. At the end of such drying stages, the result is a semi-finished ingredient of the functional food product with optimal characteristics, then sieved. A dough is made from such semi-finished ingredient, obtained through the variant (I) described above, together with extra virgin olive oil, sodium hydrogen carbonate or similar leavening agents, sugar, water, in a specified mixer. The dough is kneaded, formed, cut and then cooked at a temperature, preferably, but not limited, between 110°C and 180°C anytime between 5 and 40 minutes. However, it has to be highlighted that the cooking step can also be advantageously accomplished in a lower range, i.e. with a lower temperature between 40°C and 110°C. These conditions have been effectively established to preserve both the organoleptic and functional characteristics of the product, together with the prebiotic ability of the olive flour. Such ability has been experimentally verified in vitro, as Figure1 shows. The olive flour in fact, in an in vitro system used as a model of the human intestinal microflora activity, has the capacity to induce the growth of bifidobacteria similarly to a positive control (inulin, standard prebiotic).

Finally it has to be noted, that the food product obtained with the process of the present invention is studied in vivo in humans to verify the prebiotic characteristics

The described process allows to obtain, therefore, in the final product a good percentage of polyphenols (beneficial substances) present initially in the olive, as shown in Figure 2, which shows the polyphenols content trend one of the variant of the process (variant indicated with "II" in the flow chart).

### DEFINITIONS:

Destoning: milling the olives with a special machine (destoner) that separates the whole pits of the olives while mashing the fruit pulp.
Crushing: grinding the olive with traditional way, pulp and pits all together (can be done with a variety of machines: hammer mills, stone mills, metal tooth grinders).

## Claims

1. Process for the production of a functional food product, **characterized in that** it comprises the following operational steps:- arranging a certain quantity of olives containing a predetermined amount of polyphenols;- subjecting said quantity of olives to a grinding operation with consequently separation of the oil obtained by said grinding so as to obtain an oil-free olive paste;- subjecting said olive paste devoid of oil to a drying operation so as to obtain a dried paste;- subjecting said dried paste of olives to a grinding operation and subsequent sieving so as to obtain a semi-finished dough;- cooking said semi-finished dough so as to obtain a final product provided with optimal nutraceutical characteristics; **characterized in that** said step of drying of said paste of olives is preceded by a step of mixing of said paste of olives free of oil with flours of vegetable origin.

2. A process according to claim 1, **characterized in that** said drying step is performed under vacuum and at a temperature below 60°C.

3. A process according to claim 1, **characterized in that** said drying step is carried out at a temperature above 60°C.

4. Process according to one or more of claims 1 to 3, **characterized in that** to said semifinished dough further ingredients are added, such as extra virgin olive oil, sodium hydrogen carbonate, leavening agents, sugar, water.

5. Process according to any one of claims 1 to 4, **characterized in that** the said cooking step is preceded by a step of cutting said semifinished dough.

6. Process according to any one of claims 1 to 5, **characterized in that** said cooking step occurs at a temperature between 40 ° C and 180 °C.

7. Food product obtained with the process according to any one of the preceding claims.

## Patentansprüche

1. Prozess für die Herstellung von funktionalem Nahrungsmittelprodukt, **dadurch gekennzeichnet, dass** es die folgenden Operationen umfasst: - Bereitstellen einer gegebenen Menge der Oliven die eine vorbestimmte Menge an Polyphenolen enthalten; - Unterziehen der Olivenmenge einem Auspressvorgang mit konsequenter Trennung des aus dem Pressen erhaltenen Öls, um eine öl-freie Olivenpaste zu erhalten; - Unterwerfen der genannten öl-freien Olivenölpaste einem Trocknungsvorgang, um eine getrocknete Paste zu erhalten; - Unterwerfen der besagten getrockneten Olivenpaste einem Pressvorgang und einer nachfolgender Filterung, um einen halbfertigen Teig zu erhalten; - Kochen den besagten halbfertige, Teig, um ein Endprodukt mit ausgezeichneten Nährstoffeigenschaften zu erhalten; **dadurch gekennzeichnet, dass** dem besagten Schritt zum Trocknen der besagten Olivenpaste eine Operation zum Mischen der öl-freien Olivenpaste mit Mehl pflanzlichen Ursprungs vorausgeht.

2. Verfahren nach Anspruch 1, **dadurch gekennzeichnet, dass** der besagte Trocknungsschritt unter Vakuum und bei einer Temperatur von weniger als 60°C durchgeführt wird.

3. Verfahren nach Anspruch 1, **dadurch gekennzeichnet, dass** der besagte Trocknungsschritt bei einer Temperatur von mehr als 60°C durchgeführt wird.

4. Verfahren nach einem oder mehreren der Ansprüche 1 bis 3, **dadurch gekennzeichnet, dass** der besagten halbfertigen Paste weitere Bestandteile zugesetzt werden, wie beispielsweise extra-natives Olivenöl, Natriumhydrogencarbonat, Backtriebmittel, Zucker, Wasser.

5. Verfahren nach einem oder mehreren der Ansprüche 1 bis 4, **dadurch gekennzeichnet, dass** dem besagten Kochschritt ein Schneidvorgang der halbfertigen Paste vorausgeht.

6. Verfahren nach einem oder mehreren der Ansprüche 1 bis 5, **dadurch gekennzeichnet, dass** der Kochschritt bei einer Temperatur im Bereich von 40°C bis 180°C durchgeführt wird.

7. Nahrungsmittelprodukt, erhalten durch das Verfahren nach einem der vorhergehenden Ansprüche.

## Revendications

1. Procédé pour la production d' un produit alimentaire fonctionnel, **caractérisé en ce qu'**il comprend les opérations suivantes: - disposer une quantité donnée d'olives contenant une quantité prédéterminée de polyphénols; - soumettre ladite quantité d'olives à une opération de pressage avec séparation consécutive de l'huile obtenue à partir dudit pressage afin d'obtenir une pâte d'olive sans huile; - soumettre ladite pâte privée d'huile d'olive à une opération de séchage afin d'obtenir une pâte séchée; - soumettre ladite pâte d'olive séchée à une opération de pressage et à une filtration ultérieure afin d'obtenir une pâte semi-finie; - cuire lesdites pâtes semi-finies afin d'obtenir un produit final présentant d'excellentes caractéristiques nutritionnelles; **caractérisé en ce que** ladite opération de séchage de ladite pâte d'olive est précédée d'une opération consistant à mélanger ladite pâte d'olive sans huile avec de la farine d'origine végétale.

2. Procédé selon la revendication 1, **caractérisé en ce que** ladite opération de séchage est réalisée sous vide et à une température inférieure à 60°C.

3. Procédé selon la revendication 1, **caractérisé en ce que** ladite opération de séchage est réalisée à une température supérieure à 60°C.

4. Procédé selon une ou plusieurs des revendications 1 à 3, **caractérisé en ce que** d'autres ingrédients sont ajoutés à ladite pâte semi-finie, comme par exemple de l'huile d'olive vierge extra, de l'hydrogénocarbonate de sodium, des agents levants, du sucre, de l'eau.

5. Procédé selon une ou plusieurs des revendications 1 à 4, **caractérisé en ce que** ladite opération de cuisson est précédée d'une opération de découpe de ladite pâte semi-finie.

6. Procédé selon une ou plusieurs des revendications 1 à 5, **caractérisé en ce que** ladite opération de cuisson est effectuée à une température allant de 40°C à 180°C.

7. Produit alimentaire obtenu par le procédé selon l'une quelconque des revendications précédentes.
